(19) Europäisches Patentamt
European Patent Office
Office européen des brevets

(11) Publication number: **0 161 499**
**B1**

(12) # EUROPEAN PATENT SPECIFICATION

(45) Date of publication of patent specification: **27.07.88**

(51) Int. Cl.$^4$: **C 07 D 491/056**, A 61 K 31/47

(21) Application number: **85104478.4**

(22) Date of filing: **12.04.85**

(54) **Process for preparation of aminophthalideisoquinolines.**

(30) Priority: **17.04.84 JP 77005/84**

(43) Date of publication of application:
**21.11.85 Bulletin 85/47**

(45) Publication of the grant of the patent:
**27.07.88 Bulletin 88/30**

(84) Designated Contracting States:
**BE CH DE FR GB IT LI NL SE**

(56) References cited:
**GB-A- 873 935**

**ANNALEN DER CHEMIE, vol. 763, 1972, Verlag Chemie, Weinheim H. SUGIHARA et al. "Synthese von 2,3-Dimethoxy-5-methyl-1,4-benzochinon und seiner Äthylhomologen" pages 109-120**

(73) Proprietor: **MITSUBISHI CHEMICAL INDUSTRIES LIMITED**
**5-2, Marunouchi 2-chome Chiyoda-ku**
**Tokyo 100 (JP)**

(72) Inventor: **Takeda, Yoshiyuki**
**3-267 Nishimagari-machi Yahata-Nishi-ku**
**Kitakyushu-shi Fukuoka-ken (JP)**

(74) Representative: **Patentanwälte TER MEER - MÜLLER - STEINMEISTER**
**Mauerkircherstrasse 45**
**D-8000 München 80 (DE)**

**EP 0 161 499 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

FIELD OF THE INVENTION

The invention relates to a process for preparing the 1RS-3'RS epimer of aminophthalideisoquinolines, and more particularly, to a process for the preparation of the amino compounds from 3,4,5-trihydroxybenzoic acid as a starting material.

BACKGROUND OF THE INVENTION

Amino compounds represented by the following general formula:

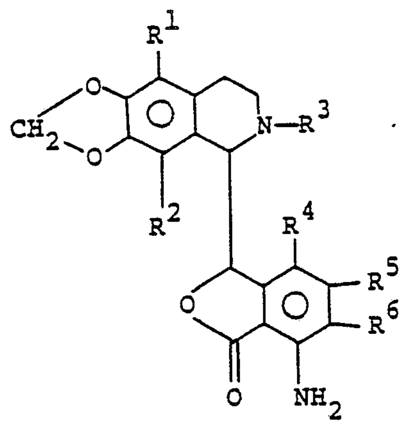

wherein $R^1$ and $R^2$ independently represent a hydrogen atom or a lower alkoxy group, $R^3$ represents a lower alkyl group, and $R^4$, $R^5$ and $R^6$ independently represent a lower alkoxy group, have two epimers, that is, 1RS-3'RS epimer (hereinafter referred to as "A-mer") and 1RS-3'SR epimer (hereinafter referred to as "B-mer"). The A-mer shows excellent properties as a drug for the treatment of liver diseases or as an anti-allergic drug.

Generally, the drug can be prepared from a trihydroxybenzoic acid as a starting material. The reaction process comprises six steps and, therefore, the yield of each step should be improved to obtain a sufficiently high total yield of the end product in order to reduce the cost of manufacture.

For example, the amino compound represented by the following formula:

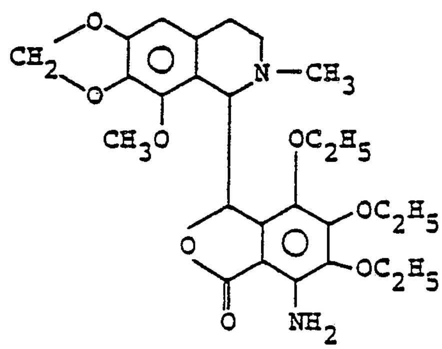

may be synthesized by the following reaction route:

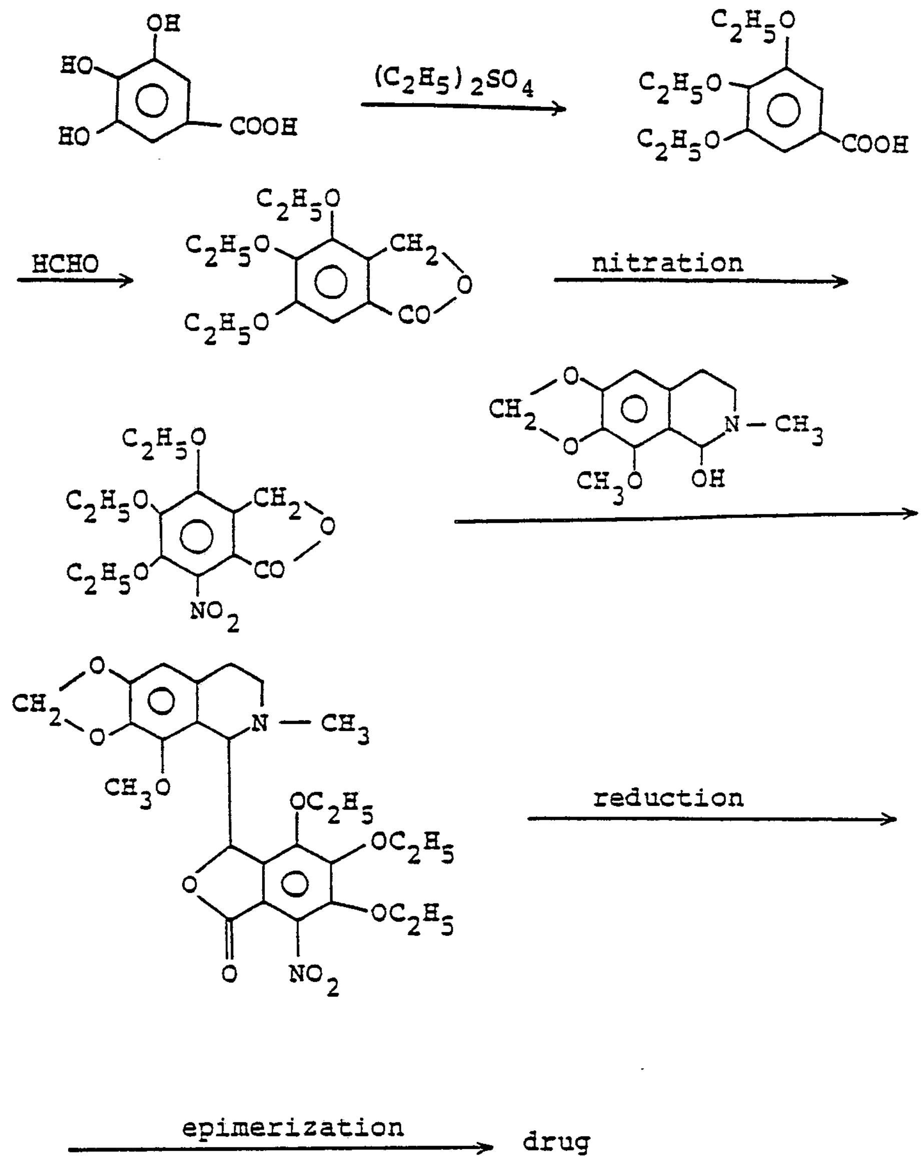


The invention provides an improvement in the step of preparation of trialkoxybenzoic acid from trihydroxybenzoic acid with dialkyl sulfate.

Generally, trialkoxybenzoic acid may be prepared by reacting trihydroxybenzoic acid with dialkyl sulfate in an aqueous caustic alkali to obtain an alkyl ester of the trialkoxybenzoic acid which is then hydrolyzed to an alkali metal salt of the trialkoxybenzoic acid, followed by acidifying the salt, as given, for example, in Ann. Chem. 763, 109—120 (1972). From page 111 of this document it is known that 3,4,5-trihydroxybenzoic acid can be reacted with dimethyl sulfate in the presence of sodium hydroxyd to provide the 3,4,5-trimethoxybenzoic acid.

The product of this process contains so much amount of by-products, that the solid mass obtained on acidifying the reaction mixture becomes gummy and very difficult to be handled. For obtaining better powdery crystals, it has been required that the acid precipitation should be carried out after refining the raw product by any means, for example, adsorption in an aqueous alkaline solution. However, there is no disclosure as to how the amount of by-products can be reduced and the handling properties of the reaction product may be improved.

It is an object of the present invention to provide an improved process for obtaining a better powdery crystal of a trialkoxybenzoic acid with a high purity in the acid precipitation of the reaction mixture.

## SUMMARY OF THE INVENTION

The present inventor has now found that the reaction of 3,4,5-trihydroxybenzoic acid with diethyl sulfate in a specified pH range effectively and significantly inhibits the formation of by-products resulting in better powdery crystals with a high yield in the acid precipitation after hydrolysis of the ethyl ester of the 3,4,5-triethoxybenzoic acid.

The subject matter of the present invention therefore is a process for preparing the 1RS-3'RS epimer of aminophthalideisoquinolines represented by the general formula (I):

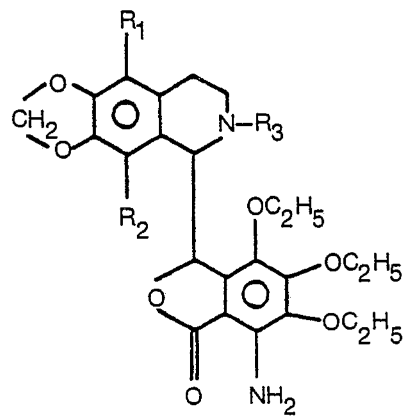

(I)

wherein $R_1$ and $R_2$ independently represent a hydrogen atom or a lower alkoxy group having one to five carbon atoms and $R_3$ is a lower alkyl group having one to five carbon atoms, comprising the steps of:

(1) reacting 3,4,5-trihydroxybenzoic acid with diethyl sulfate to produce the ethyl ester of 3,4,5-triethoxybenzoic acid which is then hydrolyzed;

(2) reacting the 3,4,5-triethoxybenzoic acid obtained in step (1) with formaldehyde;

(3) nitrating the 3,4,5-triethoxyphthalide obtained in step (2);

(4) reacting the 3,4,5-triethoxynitrophthalide obtained in step (3) with an isoquinoline represented by the general formula (III):

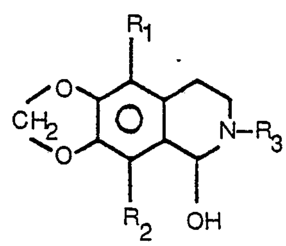

(III)

wherein $R_1$ to $R_3$ are as defined above, to produce a nitrophthalideisoquinoline represented by the general formula (II):

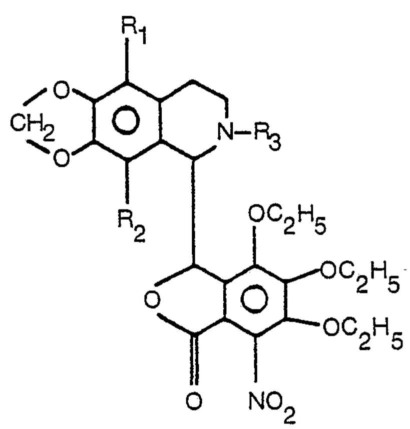

(II)

and

(5) either reducing the nitrophthalideisoquinoline followed by epimerisation or alternatively epimerizing the nitrophthalideisoquinoline followed by reduction which is characterized in that in step (1) 3,4,5-trihydroxybenzoic acid is reacted with diethyl sulfate at a temperature of from 60 to 95°C in an aqueous medium having a pH in the range of from 8.5 to 11, inclusive, maintained with the aid of a caustic alkali.

DESCRIPTION OF THE INVENTION

The invention will be fully described hereinbelow.

The "lower alkoxy or alkyl group" represented by $R^1$ to $R^3$ of the general formulae (I) to (III) includes those having one to five carbon atoms, for example, methyl, ethyl, propyl, butyl, pentyl, methoxy or ethoxy.

Preparation of trialkoxybenzoic acids

Starting materials which are used in the reaction of the invention are 3,4,5-trihydroxybenzoic acid and diethyl sulfate.

According to the invention, the reaction is carried out in an aqueous caustic alkali. The amount of the aqueous medium used is usually 4 to 10 times by weight, based on the weight of the trihydroxybenzoic acid. Caustic alkalis which may generally be used include sodium hydroxide and potassium hydroxide.

The reaction is carried out at a temperature in the range of from 60 to 95°C, preferably from 70 to 95°C. At a too low temperature the reaction rate will be too slow, in other words, the reaction cannot efficiently proceed, while, on the contrary, too high temperatures will give more undesirable by-products. The reaction period may ordinarily be about 1 to 10 hours.

The reaction may generally be performed batchwise. For example, the diethyl sulfate may be dropwise added to a base solution containing the other starting material, i.e. 3,4,5-trihydroxybenzoic acid, under reaction conditions. Usually, the diethyl sulfate may be used in an amount of 4 to 7 times by mole, preferably 4.5 to 6 times by mole, based on the amount of the trihydroxybenzoic acid. Smaller amounts of the diethyl sulfate will be unpractical since the reaction could not be completed and, therefore, intermediates or others would remain in the reaction mixture.

According to the invention, the pH of the reaction system must be maintained in the range of from 8.5 to 11, preferably from 9 to 10.5. At a pH lower than the range, the desirable 3,4,5-triethoxybenzoic acid cannot be obtained with a high yield and, moreover, only gummy mass with a low purity will be collected in the subsequent acid precipitation. On the other hand, an effective reaction cannot be expected since larger amounts of diethyl sulfate may decompose at higher pH values.

In order to maintain the pH in the predetermined range, for example, when a batchwise reaction is carried out by adding dropwise a diethyl sulfate to a base solution containing 3,4,5-trihydroxybenzoic acid, the pH can usually be adjusted by the addition of an aqueous solution of sodium hydroxide depending on the addition of the diethyl sulfate while measuring the pH of the system. Thus, the pH in the reaction system is lowered as the reaction of the dialkyl sulfate proceeds. According to the invention, the pH of the reaction system is adjusted with the aid of a caustic alkali so as to fall within the specified range.

The ethyl ester of 3,4,5-triethoxybenzoic acid produced in the abovementioned reaction is then subjected to hydrolysis to obtain an alkali salt of the trialkoxybenzoic acid. The salt is subsequently subjected to acid precipitation to collect crystals of the trialkoxybenzoic acid.

Hydrolysis is usually performed at a pH higher than 11 with a caustic alkali and at a temperature in the range of from 80 to 100°C for about 0.5 to 5 hours.

Acid precipitation is carried out until the pH of the reaction mixture is 4 or lower, preferably 3.5 or lower. Sulfuric acid or hydrochloric acid is preferably utilized.

After acid precipitation, deposited crystals may be collected by filtering the mixture according to any conventional manner. According to the present invention, the crystals are obtained in the form of powder, which can be easily handled. Filtration is ordinarily performed at 40°C or higher. Too low temperatures are not practical since inorganic salts may be deposited and contaminate the product.

The collected crystals of 3,4,5-triethoxybenzoic acid may be washed, if necessary, for example, by suspending once to three times in warm water of 30°C or higher, preferably 70°C or higher.

In one preferred embodiment of the invention, the aforementioned procedures may be performed in the following manner: 3,4,5-trihydroxybenzoic acid is charged in water and a caustic alkali is added thereto; the resulting homogeneous solution is heated under reaction conditions; simultaneously, a predetermined amount of a diethyl sulfate is fed while the pH of the reaction mixture is maintained in the specified range; after reaction, the caustic alkali is further added to increase the pH of the reaction mixture which is thus subjected to hydrolysis under heating; finally, the mixture is subjected to acid precipitation.

According to the aforementioned reaction in the invention, desirable products can be produced with an improved yield and less production of by-products, and further, powdery crystals may be collected in the subsequent acid precipitation step, by performing the reaction of 3,4,5-trihydroxybenzoic acid with diethyl sulfate at a pH in the specified range.

Preparation of 3,4,5-triethoxyphthalide

The 3,4,5-triethoxybenzoic acid obtained in the above procedures is then reacted with formaldehyde to prepare the corresponding triethoxyphthalide.

The reaction is usually carried out in an aqueous solution of an acid, such as, for example, sulfuric acid or hydrochloric acid, preferably in an aqueous solution of 5 to 40% by weight of sulfuric acid. Formaldehyde is used in the form of an aqueous solution or gas. An aqueous solution of formaldehyde, if used, may contain a small amount of a stabilizer, for example methanol. The amount of formaldehyde used is 2 to 60 times by mole, preferably 3 to 20 times by mole, based on the amount of the 3,4,5-triethoxybenzoic acid used.

The reaction may well proceed when 0.25 to 3 times by weight, preferably 0.9 to 1.4 times by weight, based on formaldehyde, of acetic acid is present.

The reaction temperature may generally be in the range of from 50 to 120°C, preferably from 70 to 110°C, and the reaction period may usually be 2 to 60 hours, preferably 4 to 30 hours. The reaction is usually carried out batchwise in a conventional reaction vessel with a stirrer.

The 3,4,5-triethoxyphthalide produced in the reaction is collected from the mixture. Generally, the reaction mixture is cooled to 0 to 50°C and the deposited crystals are filtered out. The crystals are then washed with an aqueous alkaline solution to remove unreacted materials contained in the crystals.

An aqueous alkaline solution which may be used is an aqueous solution of 0.5 to 30% by weight, preferably 1 to 5% by weight, of e.g. solium hydroxide, potassium hydroxide, sodium carbonate or ammonia. Washing with an alkali is usually performed by suspension under conditions, for example, at 60 to 10°C for 0.5 to 3 hours.

Preparation of 3,4,5-triethoxynitrophthalide

The 3,4,5-triethoxyphthalide is then subjected to nitration to obtain the 3,4,5-triethoxynitrophthalide.

One example of nitration procedures which may generally be utilized is a method using a nitrate salt, such as cupric nitrate and zinc nitrate, but the invention is not limited to this method.

In such a method, there may generally be utilized, as a solvent, an acid anhydride, such as acetic anhydride, trifluoroacetic anhydride and propionic anhydride, or a lower fatty acid, such as acetic acid and trifluoroacetic acid. In combination with such a solvent, there may also be used an inert solvent, such as haloalkanes, for example, dichloromethane, carbon tetrachloride or dichloroethane, nitroalkanes, for example, nitromethane or nitroethane, and nitrobenzene. The solvent may usually be used in an amount of 2 to 20 times by weight, preferably 3 to 6 times by weight, based on the amount of the 3,4,5-triethoxyphthalide used.

The nitration reaction is generally carried out at 0 to 120°C, preferably 20 to 60°C, for 0.5 to 4 hours. The amount of a nitrating agent used, namely nitrate salt, is generally 0.8 to 5 times by mole, preferably 1 to 2 times by mole, based on the amount of the 3,4,5-triethoxyphthalide used.

After reaction, the 3,4,5-triethoxynitrophthalide produced is usually deposited by adding water to the reaction mixture followed by filtration to collect the crystals.

Other methods, for example, utilizing a mixed acid of nitric acid and sulfuric acid as a nitrating agent can also be used in the nitration step of the invention.

Preparation of nitrophthalideisoquinolines

The trialkylnitrophthalide is then subjected to condensation reaction with an isoquinoline represented by the following general formula (III):

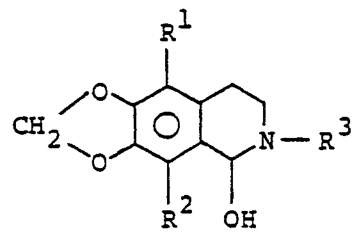

(III)

wherein $R^1$ to $R^3$ are as defined above, to prepare a nitrophthalideisoquinoline represented by the following general formula (II):

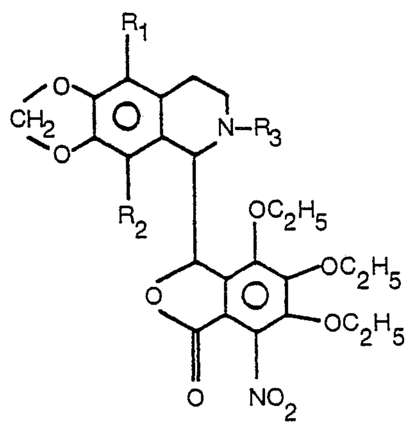

(II)

wherein $R^1$ to $R^3$ are as defined above.

The condensation is generally carried out at a temperature in the range of from 20 to 100°C, preferably from 40 to 80°C, for approximately 1 to 3 hours in an aliphatic alcohol as a solvent, for example, methanol, ethanol, propanol or butanol. The reaction rate will unsuitably be slow at a lower temperature, while undesirable decomposition of the starting material, i.e. isoquinoline, may occur if the temperature is too high.

In the condensation reaction, either A-mer or B-mer of the product, i.e. nitrophthalideisoquinoline, can be produced, depending on the difference in the configuration, RS or SR, of hydrogen atom at the condensing position, that is, of hydrogen atom bonded to the carbon atom at 1-position of the isoquinoline or 3′-position of the nitrophthalide. Thus, there may usually be obtained a mixture of A-mer and B-mer of the product. Generally, the longer is the reaction period, the higher is the ratio of A-mer but the lower is the yield of the product. In the present invention, however, the proportion of A-mer in the product produced in this condensation step is not especially limited, since the produced B-mer can be well converted into A-mer in the later step described hereinbelow. The proportion (content) of A-mer in the product may usually be in the range of approximately 20 to 80% by mole.

The amount of the isoquinoline used may generally be in the range of from 0.8 to 1.2 times by mole, preferably 0.9 to 1.0 times by mole, based on the amount of the nitrophthalide used. Smaller amount results in a low yield of the product, i.e. nitrophthalideisoquinoline, while an amount more than the upper limit may cause undesirable decomposition of the isoquinoline.

Generally, the crystalline product can easily be collected by crystallization by cooling or water dilution of the reaction mixture at the end of this condensation step.

Reduction and epimerization

The nitrophthalideisoquinoline is further treated to prepare A-mer of the amino compound represented by the general formula (I), which is useful for a drug, by either (A) reduction followed by epimerization or (B) epimerization followed by reduction. The procedures (A) are more preferred since A-mer of the amino compound may be obtained with a higher yield.

Reduction methods which may be utilized in the invention include: (i) reduction using a metal borohydride as a reducing agent, such as $NaBH_4$, $LiBH_4$, $NaBH_3CN$, $NaBH_2S_3$, $NaBH(OCH_3)_3$, $NaBH_3(OH)$ or $KBH_4$, in the presence of a catalyst selected from the group consisting of metals of the IB and VIII groups, such as Cu, Ag, Ni, Pd and Rh, and salts thereof, such as hydrochlorides, sulfates and acetates; (ii) reduction using as a reducing agent a combination of a metal, such as Sn, Fe or Zn, or a compound thereof with hydrochloric acid; (iii) catalytic hydrogenation using a platinum group metal, such as Pd and Pt, or a compound thereof as a catalyst; and (iv) reduction using $LiBH_4$ or $NaBH_2S_3$ as a reducing agent in the absence of any catalyst.

These reduction procedures are generally carried out at a temperature in the range of from 0 to 100°C, preferably from 20 to 60°C, although the temperature can slightly value depending on each procedure. Higher temperatures may yield a larger amount of by-products. On the contrary, a lower temperature will result in a slower reaction rate and, therefore, the desired amino compounds cannot efficiently be obtained. The reaction period is usually 10 minutes to 4 hours.

The reduction may generally be carried out in a solvent which may slightly vary depending on each procedure, for example, an aliphatic alcohol, such as methanol, ethanol and propanol, an ether, such as diglyme and tetrahydrofuran, an aliphatic ketone, such as acetone and methyl ethyl ketone, a water-miscible organic solvent, such as dimethylformamide and dimethyl sulfoxide, an aqueous solution thereof. When a metal borohydride is used as a reducing agent, an aliphatic alcohol may be particularly preferred since the mixture can immediately be subjected to the subsequent epimerization step described below. The solvent may generally be used in an amount of 2 to 50 times by weight, preferably 3 to 20 times by weight, based on the nitrophthalideisoquinoline of the general formula (II) used. The nitrophthalideisoquinolines may also be supplied in the form of a solution in e.g. acetone or acetic acid.

The amino compound of the general formula (I) can be separated and collected from the reduced mixtures: for example, the reaction mixture can be subjected to extraction at a neutral or alkaline pH with an organic solvent in which the amino compound can be dissolved, such as halogenated hydrocarbons. Thus, the amino compound can be collected in an organic phase. When any solid catalyst is present in the reaction mixture, the solid may preliminarily be either filtered out or dissolved, if necessary.

The epimerization of the aminophthalideisoquinoline can generally be performed in an aliphatic alcohol in the presence of an alkali, while the epimerization of the nitrophthalideisoquinoline, according to the procedure (B) given above, can be performed by heating without alkali in the same solvent.

The aliphatic alcohols which may be used include those having 1 to 5 carbon atoms, such as methanol, ethanol, propanol and butanol. The solvent may also contain some water. The solvent may usually be used in an amount of 3 to 20 times by weight based on the amount of the amino compound of the general formula (I) used.

The alkali which may be used is, for example, an alkali hydroxide, such as sodium hydroxide, potassium hydroxide and barium hydroxide, an alkali alkoxide, such as sodium alcoholate. The alkali may be used in a concentration of 1 to 5% by weight, preferably 2 to 5% by weight. Lower concentrations will cause a slow reaction rate while higher concentrations may result in a low yield. The amount of the alkali used is 0.4 to 3 times by mole, preferably 0.9 to 2 times by mole, based on the amount of B-mer to be

epimerized.

The epimerization may generally be performed at a temperature in the range of from 20 to 100°C, preferably from 50 to 80°C. Lower or higher temperatures cannot result in a desirably high content of A-mer. The epimerization period may vary depending on the temperature and the concentration of alkali used, but is generally 2 to 20 hours.

The epimerization of the amino compound may generally be carried out by adding the material into a solvent containing a predetermined amount of an alkali and treating the mixture under predetermined conditions with stirring. In these conditions, although B-mer can be dissolved, A-mer may hardly be dissolved and remains as crystals. Therefore, the mixture can be treated either directly in the form of a slurry or after preliminarily separating the precipitated A-mer. Further, since the epimerized mixture contains both the deposited A-mer and the dissolved B-mer, the desired crystal of A-mer can be obtained by solid-liquid separation of the mixture.

The A-mer of the amino compound can be recovered with a high purity by a simple filtration of the mixture after the epimerization. Thus, when the epimerization is performed after reduction, a highly pure A-mer of the amino compound of the general formula (I), which may be used as a bulk of a drug, can directly be collected from the mixture after the procedures.

According to the present invention, compounds which are useful for a drug for the treatment of liver diseases or allergies can be prepared advantageously and industrially from a trihydroxybenzoic acid.

## DESCRIPTION OF THE PREFERRED EMBODIMENTS

The invention will be fully and clearly illustrated with the following examples.

### Example 1

Into a 30 liter reaction vessel provided with a stirrer and a heater, 925 g (4.916 mole) of 3,4,5-trihydroxybenzoic acid and 6,000 g of water were charged. After purging by nitrogen gas, 2,060 g (12.86 mole of NaOH) of a 25% aqueous solution of sodium hydroxide was added at a temperature of 15°C or lower under stirring to completely dissolve 3,4,5-trihydroxybenzoic acid. The resulting mixture had a pH of 10.5.

After 1,060 g (6.88 mole) of diethyl sulfate was added at a temperature of 15°C or lower, the mixture was heated up to 80°C over a time period of 30 minutes while adding a 25% aqueous solution of sodium hydroxide so that the pH of the mixture was maintained at 9 to 9.5. Then, 2,835 g (18.39 mole) of diethyl sulfate and 2,750 g of a 25% aqueous solution of sodium hydroxide (17.17 mole of NaOH) were separately added dropwise at 80 to 90°C over one hour so that the pH was maintained at 9 to 9.5. Thereafter, the mixture was reacted at 90°C for an additional half one hour.

After the reaction time of 30 minutes, 2,560 g of a 25% aqueous solution of sodium hydroxide (15.98 mole of NaOH) was added to the reaction mixture, so that the pH was adjusted up to 11.5. The mixture was hydrolyzed under reflux at 90 to 95°C for 3 hours. After hydrolysis, 2,000 g of 50% sulfuric acid (10.2 mole of $H_2SO_4$) was added at 70°C so that the pH was adjusted to 3 for acid precipitation. Deposited crystals were filtered at 40°C, washed with water, filtered again and dried to collect the crystal of 3,4,5-triethoxybenzoic acid.

The yield based on 3,4,5-trihydroxybenzoic acid and the purity of the obtained crystal are shown in Table 1 below.

### Comparative Examples 1 and 2

In the procedures of Example 1, the addition rate of an aqueous solution of sodium hydroxide was changed so that the pH of the reaction mixture was maintained at a value shown in Table 1 in the reaction of 3,4,5-trihydroxybenzoic acid with diethyl sulfate. The results are shown in Table 1.

Table 1

| | pH Range | Yield (%) | Purity (%) | Crystal Form |
|---|---|---|---|---|
| Example 1 | 9 - 9.5 | 90 | 95 | Powder |
| Comparative Example 1 | 3 | 48 | 55 | Gummy |
| Comparative Example 2 | 7.3-8.0 | 68 | 68 | Gummy |

Examples 2—4

The procedures of Example 1 were repeated except that a pH range was changed as shown in Table 2. The results are shown in Table 2.

Table 2

| | pH Range | Yield (%) | Purity (%) | Crystal Form |
|---|---|---|---|---|
| Example 2 | 10.0-10.4 | 92 | 96 | Powder |
| Example 3 | 10.5-10.8 | 89 | 93 | Powder |
| Example 4 | 8.5-8.7 | 80 | 83 | Powder |

Example 5

Preparation of aminophthalideisoquinoline

Tritoqualine which is included in the amino compounds of the general formula (I) was prepared from 3,4,5-triethoxybenzoic acid produced in Example 1.

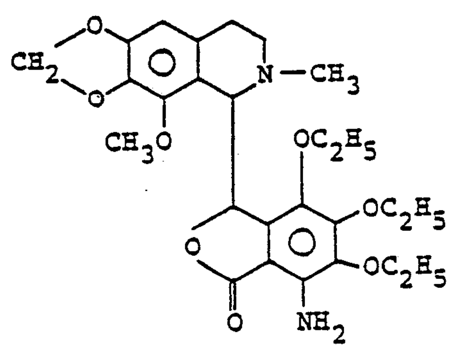

Preparation of 4,5,6-triethoxyphthalide

Into a 2 liter reaction vessel provided with a stirrer and a heater, there were charged 203.5 g (0.8 mole) of 3,4,5-triethoxybenzoic acid, 519 g (6.39 mole of HCHO) of an aqueous solution of 37% by weight of formaldehyde, 204.1 g of 98% by weight of sulfuric acid, 211 g of glacial acetic acid and 469 g of water, and the mixture was reacted at 95°C under stirring for 14 hours.

After reaction, the reaction mixture was cooled to 40°C to solidify the oily components in the mixture. After the deposited cakes were filtered and washed with water, the cakes were washed by suspending in 800 ml of an aqueous solution of 3% by weight of ammonia at 30°C for one hour. Suspension washing was further carried out at 30°C for one hour in 600 ml of an aqueous solution of 80% by volume of methanol. After filtration and drying there was obtained 125.8 g of 4,5,6-triethoxyphthalide.

High performance liquid chromatography showed a purity of 99% and a yield of 59% of the crystal.

Preparation of 4,5,6-triethoxy-7-nitrophthalide

There was added 53.2 g (0.20 mole) of 4,5,6-triethoxyphthalide into 204 g of acetic anhydride. After the mixture was charged in a reaction vessel, 67.6 g (0.28 mole) of cupric nitrate trihydrate was added over one hour under stirring while maintaining the temperature of the reaction mixture at 30°C. After the addition, the reaction mixture was stirred at 30°C for one hour and then poured into 800 g of ice water. The mixture was stirred at room temperature for one hour. Deposited crystals were filtered out, thoroughly washed with water, and dissolved in 300 ml of methanol under heating at 40°C to remove insolubles. After distilling out 240 ml of methanol from the filtrate under reduced pressure, 30 ml of water was added to the residual solution. The deposited crystals were filtered out and dried. There was obtained 40.4 g of 4,5,6-triethoxy-7-nitrophthalide with a yield of 65%.

Preparation of 2-methyl-6,7-methylenedioxy-8-methoxy-1-[4,5,6-triethoxy-7-nitrophthalidyl-(3)]-1,2,3,4-tetrahydroisoquinoline

Into a glass reaction vessel, there were charged 31.1 g (0.1 mole) of 4,5,6-triethoxy-7-nitrophthalide, 23.7 g (0.1 mole) of cotarnine and 80 ml of methanol. Condensation was carried out under stirring at 60°C for 2 hours.

After reaction, 50 ml of water was added to the reaction mixture and cooled at 20°C. There was deposited the end product represented by the following formula:

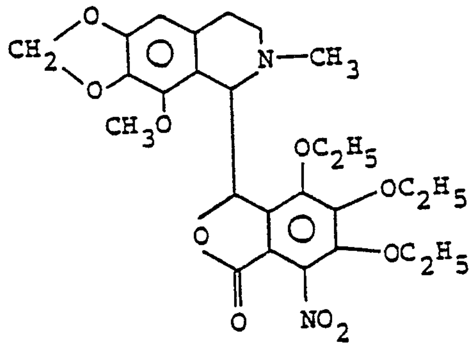

There was obtained the product with a yield of 75% based on cotarnine. The product contained 46% of A-mer and 54% of B-mer.

Preparation of tritoqualine

Into a glass reaction vessel, 26.5 g (0.05 mole) of the product obtained above, 1.76 g of cupric sulfate and 80 ml of methanol were charged, and a solution of 3.8 g (0.1 mole) of sodium borohydride in 35 ml of methanolic solution of 1N sodium hydroxide was dropwise added at 35°C under stirring over one hour. Stirring was continued at the same temperature for an additional one hour to react. Analysis of the product in the resultant mixture showed the conversion rate of 98% and selectivity of 98%.

After adding 50 ml of 35% hydrochloric acid to the mixture obtained above in order to decompose the remaining excess sodium borohydride, copper was oxidized by blowing air thereinto. Then, 60 ml of 28% aqueous ammonia and 50 ml of water were added to convert the amino compound into the free form, while copper formed an amine complex with ammonia. The mixture was then extracted with 160 ml of dichloromethane. Thus, the amino compound was well extracted into the organic phase, while the metal components remained in the water layer, not giving any deposit. In these procedures, methanol was totally distributed into the water layer while the product was recovered into the organic phase with a yield of approximately 100%.

To the obtained solution of the amino compound in dichloromethane, 480 ml of methanol was added and the solvent was distilled off to concentrate to the solvent volume of 100 ml. Thus, methanol was substituted for dichloromethane.

Epimerization

Into a glass reaction vessel, the obtained methanol slurry containing the amino compound (A-mer content of 46% and B-mer content of 54%) and 4.4 g of sodium hydroxide were charged. After the reaction was carried out at 60°C under stirring for 10 hours, the mixture was filtered to recover the crystal of A-mer.

The resulting reaction mixture contained 23.0 g (content of 96.6%) of A-mer and 0.8 g (content of 3.4%) of B-mer. The solid product obtained by filtration had a purity of about 100%, and the yield of the A-mer was 91% based on the nitro compound used as a starting material.

Thus, tritoqualine (A-mer) which is useful for a drug can be manufactured advantageously and industrially from the trihydroxybenzoic acid as a starting material.

**Claims**

1. A process for preparing the 1RS-3'RS epimer of aminophthalideisoquinolines represented by the general formula (I):

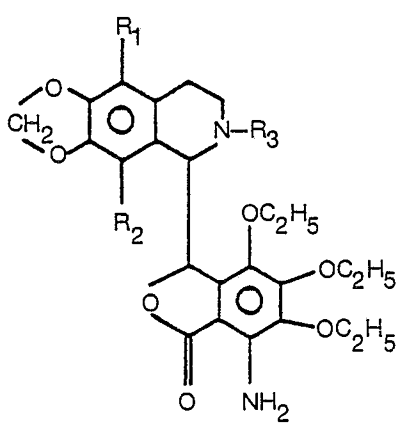

(I)

wherein $R_1$ and $R_2$ independently represent a hydrogen atom or a lower alkoxy group having one to five carbon atoms and $R_3$ is a lower alkyl group having one to five carbon atoms, comprising the steps of:

(1) reacting 3,4,5-trihydroxybenzoic acid with diethyl sulfate to produce the ethyl ester of 3,4,5-triethoxybenzoic acid which is then hydrolyzed;

(2) reacting the 3,4,5-triethoxybenzoic acid obtained in step (1) with formaldehyde;

(3) nitrating the 3,4,5-triethoxyphthalide obtained in step (2);

(4) reacting the 3,4,5-triethoxynitrophthalide obtained in step (3) with an isoquinoline represented by the general formula (III):

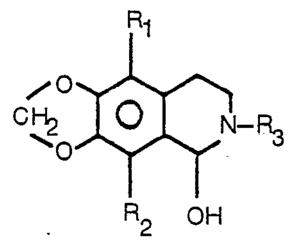

(III)

wherein $R_1$ to $R_3$ are as defined above, to produce a nitrophthalideisoquinoline represented by the general formula (II):

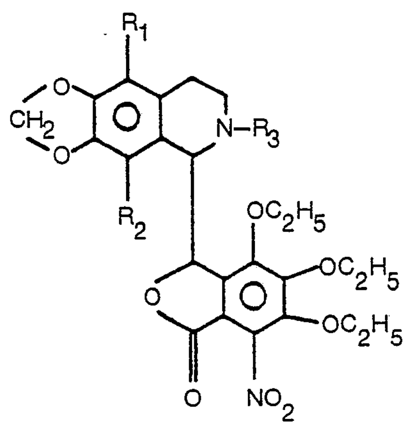

(II)

and

(5) either reducing the nitrophthalideisoquinoline followed by epimerisation or alternatively epimerizing the nitrophthalideisoquinoline followed by reduction; characterized in that in step (1) 3,4,5-trihydroxybenzoic acid is reacted with diethyl sulfate at a temperature of from 60 to 95°C in an aqueous medium having a pH in the range of from 8.5 to 11, inclusive, maintained with the aid of a caustic alkali.

2. The process as defined in claim 1, characterized in that the hydrolysis of the ethyl ester of 3,4,5-triethoxybenzoic acid in step (1) is carried out at a pH of 11 or higher.

3. The process as defined in claim 1, characterized in that the reaction mixture after the hydrolysis in step (1) is subjected to acid precipitation at a pH of 4 or lower to collect crystals of the 3,4,5-triethoxybenzoic acid.

**Patentansprüche**

1. Verfahren zur Herstellung des 1RS-3'RS-Aminophthalidisochinolinepimeren der allgemeinen Formel (I):

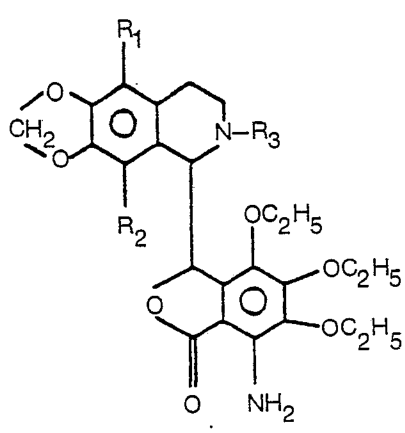

(I)

worin $R_1$ und $R_2$ voneinander unabhängig Wasserstoffatome oder Niedrigalkoxygruppen mit 1 bis 5 Kohlenstoffatomen und $R_3$ eine Niedrigalkylgruppe mit 1 bis 5 Kohlenstoffatomen bedeuten, durch

(1) Umsetzen von 3,4,5-Trihydroxybenzoesäure mit Diethylsulfat unter Bildung des 3,4,5-Triethoxybenzoesäureethylesters, der anschließend hydrolysiert wird;

(2) Umsetzen der in Stufe (1) erhaltenen 3,4,5-Triethoxybenzoešaure mit Formaldehyd;

(3) Nitrieren des in Stufe (2) erhaltenen 3,4,5-Triethoxyphthalids;

(4) Umsetzen des in Stufe (3) erhaltenen 3,4,5-Triethoxynitrophthalids mit einem Isochinolin der allgemeinen Formel (III):

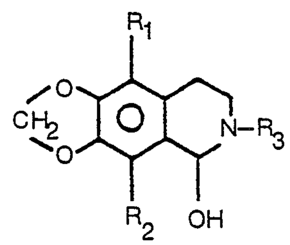

(III)

worin $R_1$ bis $R_3$ die obigen Bedeutungen haben, unter Bildung eines Nitrophthalidisochinolins der allgemeinen Formel (II):

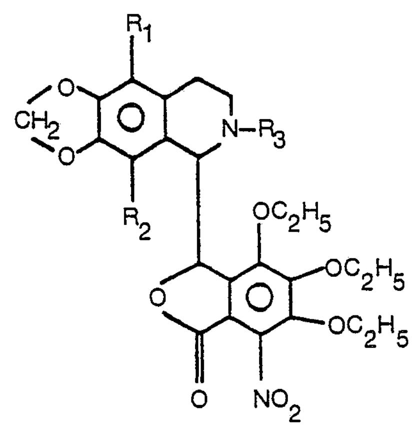

(II)

und

(5) Reduzieren des Nitrophthalidisochinolins und anschließende Epimerisierung oder Epimerisierung des Nitrophthalidisochinolins und anschließende Reduktion als Alternative,

dadurch gekennzeichnet, daß man in Stufe (1) die 3,4,5-Trihydroxybenzoesäure mit Diethylsulfat bei einer Temperatur von 60 bis 95°C in einem wäßrigen Medium mit einem mit Hilfe eines Ätzalkalis aufrechterhaltenen pH-Wert im Bereich von 8,5 bis einschließlich 11 umsetzt.

2. Verfahren nach Anspruch 1, dadurch gekennzeichnet, daß man die Hydrolyse des 3,4,5-Triethoxybenzoesäureethylesters in Stufe (1) bei einem pH-Wert von 11 oder höher durchführt.

3. Verfahren nach Anspruch 1, dadurch gekennzeichnet, daß man die Reaktionsmischung nach der Hydrolyse in Stufe (1) einer Säurefällung bei einem pH-Wert von 4 oder weniger zum Sammeln der 3,4,5-Triethoxybenzoesäurekristalle unterwirft.

**Revendications**

1. Procédé de préparation de l'épimère 1RS-3'RS des aminophtalideisoquinoléines représenté par la formule générale (I):

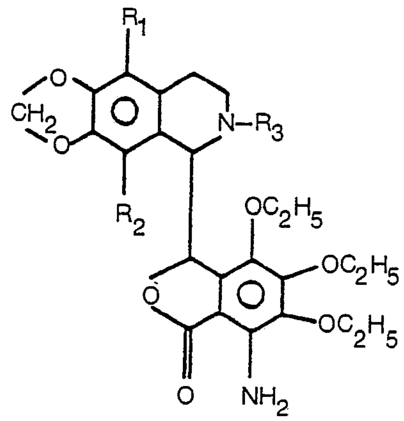

(I)

où $R_1$ et $R_2$ représentent indépendamment un atome d'hydrogène ou un groupe alcoxy inférieur comportant de un à cinq atomes de carbone et $R_3$ est un groupe alcoyle inférieur comportant de un à cinq atomes de carbone, comprenant les étapes de:

(1) réaction de l'acide 3,4,5-trihydroxybenzoïque avec du sulfate de diéthyle pour produire l'ester éthylique de l'acide 3,4,5-triéthoxybenzoïque que l'on hydrolyse alors;

(2) réaction de l'acide 3,4,5-triéthoxybenzoïque obtenu dans l'étape (1) avec du formaldéhyde;

(3) nitration du 3,4,5-triéthoxyphtalide obtenu dans l'étape (2);

(4) réaction du 3,4,5-triéthoxynitrophtalide obtenu dans l'étape (3) avec une isoquinoléine représentée par la formule générale (III):

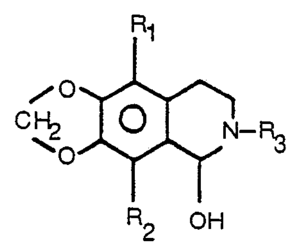

(III)

où $R_1$ à $R_3$ sont tels que définis ci-dessus, pour produire une nitrophtalideisoquinoléine représentée par la formule générale (II):

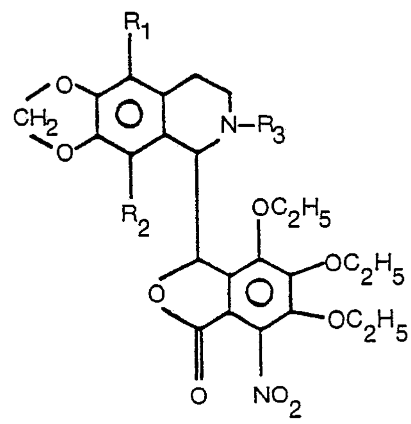

(II)

et

(5) soit réduction de la nitrophtalideisoquinoléine suivie par une épimérisation, soit encore épimérisation de la nitrophtalideisoquinoléine suivie par une réduction; caractérisé en ce que dans l'étape (1) on fait réagir l'acide 3,4,5-trihydroxybenzoïque avec du sulfate de diéthyle à une température allant de 60 à 95°C dans un milieu aqueux ayant un pH situé dans l'intervalle allant de 8,5 à 11, compris, maintenu à l'aide d'une base caustique.

2. Procédé tel que défini dans la revendication 1, caractérisé en ce que l'hydrolyse de l'ester éthylique de l'acide 3,4,5-triéthoxybenzoïque dans l'étape (1) est conduite à un pH égal ou supérieur à 11.

3. Procédé tel que défini dans la revendication 1, caractérisé en ce qu'on soumet le mélange réactionnel après l'hydrolyse dans l'étape (1) à une précipitation acide à un pH égal ou inférieur à 4 pour recueillir des cristaux de l'acide 3,4,5-triéthoxybenzoïque.